# EUROPEAN PATENT APPLICATION

(11) **EP 0 659 879 A2**
(43) Date of publication of application: **28.06.1995**
(21) Application number: 94309575.2
(22) Date of filing: 20.12.1994
(51) Int. Cl.: C12N 1/20, A61K 39/10

(54) **Control of pH during bordetella growth**

(30) Priority: 22.12.1993 US 171395
(71) Applicant: CONNAUGHT LABORATORIES LIMITED, Willowdale Ontario M2R 3T4 (CA)
(72) Inventor: Herbert, Andrew, Toronto, Ontario M5P 2H9 (CA); Lutzen, Niels, Boston, MA 01701 (US); Kole, Manoj, Willowdale, Ontario M2R 2W4 (CA)
(74) Representative: Smart, Peter John

(57) **Abstract**

Bordetella species, such as Bordetella pertussis, is grown under aerobic conditions in a liquid culture medium containing nutrients for such growth. Enhanced cell density and antigen production are achieved by continuously determining the pH of the culture medium and, in response to such determination, simultaneously feeding, as required, (a) an acid, usually a mineral acid, such as hydrochloric acid, phosphoric acid or sulphuric acid, in a concentration sufficient to maintain the pH of the culture medium within a predetermined range of values, usually about pH 6.8 to about pH 7.3, and (b) a nutrient medium containing organic carbon and organic nitrogen, usually a neutral salt of an amino acid, such as monosodium glutamate, an amino acid, such as glutamic acid, or a mixture thereof.

## Description

### FIELD OF INVENTION

The present invention is directed to a method for the control of pH during the growth of a Bordetella organism, particularly Bordetella pertussis, which also provides nutrients for Bordetella growth to the culture medium, resulting in improved production of materials used in pertussis vaccines.

### BACKGROUND TO THE INVENTION

The severe respiratory disease pertussis (whooping cough) is caused by localized infection by Bordetella pertussis. A common vaccine against such disease comprises inactivated Bordetella pertussis. The organism is grown in a culture medium and then inactivated by formaldehyde to provide the inactivated B. pertussis.

The initial culture medium for Bordetella growth is made up from sources of organic carbon and organic nitrogen, as well as inorganic salts, vitamins and amino acids. Materials often used to provide such sources include casamino acid, sodium glutamate, proline, cysteine and glutamic acid. Following adjustment to the desired pH, cultures of the organism are grown under aerobic conditions to a desired cell density.

It is known to effect control of the pH of the culture medium within a desired range during the fermentation by employing acid or alkali, as necessary. In general, the fermentation medium tends to drift towards more alkaline values during the fermentation and hence pH adjustment usually is effected using acid during the fermentation operation.

### SUMMARY OF INVENTION

In accordance with the present invention, an improved procedure is provided for the batch fermentation of Bordetella organisms, particularly Bordetella pertussis, under aerobic conditions to high cell density and high antigen production.

The batch fermentation is effected in a liquid culture medium containing nutrients for growth of the organism, usually a source of carbon, nitrogen, salts and vitamins. The pH of the culture medium is continuously determined during growth of the organism and, in response to such determination, there is simultaneously fed to the culture medium, as required, (a) an acid in a concentration sufficient to maintain the pH of the culture medium within a predetermined range of values during growth of the organism and (b) a nutrient medium for cell growth containing organic carbon and organic nitrogen.

By effecting such simultaneous feed of acid and nutrients to the culture medium in response to changes in pH, high cell density and antigen production are achieved. Since changes in pH result from cell growth, the process of the invention is self-limiting with respect to cell growth during the fermentation procedure.

### GENERAL DESCRIPTION OF INVENTION

The range of pH within which the culture medium is controlled throughout the fermentation generally is from about 6.0 to about 9.0, preferably about 6.8 to about 7.3. The determination of pH and, as necessary, adjustment preferably is effected automatically, using a pH meter operatively connected to a pump, which may be a peristaltic pump, for pumping acid to the culture medium in response to pH determinations outside a predetermined range.

The acid employed in achieving the pH control during the fermentation procedure of the invention is generally a mineral acid, usually hydrochloric acid, phosphoric acid or sulfuric acid. The nutrient medium employed in the present invention generally comprises a neutral salt of an amino acid, an amino acid or a mixture of the two. One suitable neutral salt of an amino acid is monosodium glutamate while one suitable amino acid is glutamic acid. Other suitable neutral salts of an amino acid which may be employed herein include monopotassium glutamate, monopotassium aspertate and monosodium aspertate. Other suitable amino acids include glutamic acid hydrochloride, L-serine, L-proline and L-lysine hydrochloride.

The simultaneous feed of the acid and the nutrient medium in the process of the invention generally is achieved by providing a solution of the nutrient medium in the acid. This solution then is fed, as required, to the culture medium, in response to the pH determination. In general, the concentration of nutrients provided in the acid should be sufficient to permit growth of the organism and is dependent on the nutrient and acid chosen. One convenient form of feed solution is a solution of 2 M glutamic acid in 1 M HCl. Other nutrients also may be present in the solution fed to the culture medium. The employment of a solution of the amino acid in the mineral acid permits a single feed of additives to the cell culture to be effected during the fermentation operation.

By feeding nutrients in response to pH change in accordance with the invention, the fermentation is self-limiting. Nevertheless, the fermentation procedure is generally of shorter duration than a fermentation procedure in which nutrients are added separately and, after an initial fermentation period, continuously to the culture medium independent of acid addition, with greater production of pertussis toxin and other pertussis antigens by the cell mass.

The fermentation process of the invention may be employed in a procedure to form an acellular Bordetella pertussis vaccine comprising B. pertussis antigens produced by the cell mass or a detoxified whole cell B. pertussis vaccine by inactivating the cell mass produced.

### EXAMPLES

### Example 1

This Example demonstrates the production of pertussis antigens using a fermentation procedure according to the present invention.

The organism used in this Example was the Connaught Laboratories Limited production strain of Bordetella pertussis, strain 10536. A modified Stainer-Scholte medium was used for the fermentation comprising:

| Component | mg/L |
|---|---|
| NaCl | 2,500 |
| KH₂PO₄ | 500 |
| KCl | 200 |
| MgCl₂.6H₂O | 100 |
| Tris base | 1,500 |
| Casamino acid | 10,000 |
| Monosodium glutamate | 10,000 |

The pH of the medium was adjusted to 7.0 with HCl and the medium autoclaved at 120°C for 30 minutes.

A growth factor solution also was used for fermentation, comprising:

| Component | mg/L |
|---|---|
| L-cysteine HCl | 40 |
| Nicotinic acid | 4 |
| Ascorbic acid | 400 |
| Glutathione, reduced | 150 |
| FeSO₄.7H₂O | 10 |
| Heptakis-(2-6-0-dimethyl)-β-cyclodextrin | 1000 |
| CaCl₂.2H₂O | 20 |

The growth factor solution was filter sterilized and added to cool media in the fermentor at a ratio of 10 mL growth factors per litre of main medium.

A seed vial of B. pertussis strain 10536 was revived on agar medium (modified Stainer-Scholte medium containing 1.5% agar). Growth from the agar was transferred to 4 litre Erlenmeyer flasks, each containing one litre of the modified Stainer-Scholte medium described above. The flasks were placed on a rotary shaker and agitated at 150 rpm and 36°C. The culture was used to inoculate the fermentor before the end of the exponential phase (1 to 2 g/L dry weight cell concentration).

The fermentation was performed in a 14-litre Chemap fermentor containing 10 L of modified Stainer-Scholte medium. The fermentor was sterilized at 121°C for 30 minutes. The pH of the fermentation broth was continuously measured by an Ingold sterilizable pH electrode and maintained in the pH range of 7.0 to 7.3 during the fermentation.

The pH was maintained in this predetermined range by adding a solution of dilute hydrochloric acid containing glutamic acid (2.0 M glutamic acid in 1 M HCl) to the fermentor by the on-off activation of a peristaltic pump as required by the pH measurements.

A sterilizable polarographic oxygen electrode and a dissolved oxygen controlled system were used to monitor and control the oxygen level in the fermentor at 30% of air saturation.

During fermentation, samples were taken at different intervals. Bacterial concentration was determined spectophotometrically (O.D.₆₀₀) and by direct weight measurement. Final fermentor samples were analyzed for soluble PT (LPF), FHA and 69Kd antigens using enzyme-linked immunoassays (ELISAs).

The results for a series of such fermentations are shown in Table I below. As may be seen from the results shown in Table I, the growth was consistent in these experiments. The average growth for the four experiments was O.D.₆₀₀ = 7.49. The PT antigen production varied from approximately 30 to 40 mg/L with an average for the four experiments of 32.37 mg/L. FHA antigen production was 60 to 100 mg/L in the experiments, with an average production of 83.33 mg/L. 69Kd antigen production varied from 7 to 10 mg/L with an average production of 8.75 mg/L.

The results shown in Table I demonstrate that the pH control method of the invention results in high cell and antigen production.

### Example 2

In this Example, fermentations carried out by the method described in Example 1 are compared with fermentation carried out using a mineral acid for pH control and a supplemental separate nutrient feed during the growth phase (Fed-batch method).

The difference between the Fed-batch method and the method described in Example 1 are:
1. pH was controlled automatically by adding 2.5 M phosphoric acid to the fermentor by the on-off activation of a peristaltic pump and using a standard pH electrode and pH controller.
2. A feed supplement was added when the initial batch nutrients were exhausted. The nutrient supplement had the following composition.

| Component | mg/L |
|---|---|
| Monosodium glutamate | 400,000 |
| L-cysteine HCl | 2,000 |
| Nicotinic acid | 200 |
| Ascorbic acid | 20,000 |
| Glutathione (reduced) | 7,600 |
| FeSO₄.7H₂O | 500 |

The supplement was filter sterilized and added slowly and continuously to the fermentor when the initial batch of nutrients was exhausted by a feed pipe separate from that feeding the acid to the fermentor. The results obtained from the fermentation are set forth in Table II below.

The results shown in Table II compare the productivity of the Fed-batch method and the method described in Example 1. The method described in Example 1 increased PT production by 30% and 69Kd production by 41% in comparison to the Fed-batch method. The method of Example 1, however, decreased FHA production by 64%. The method described in Example 1 saves 20 to 25% of fermentation time. Biomass production in the Fed-batch method was increased by 25%.

### Example 3

This Example illustrates the production of whole cell pertussis vaccine using the fermentation procedure of the invention.

A modified Stainer-Scholte medium was used for fermentation, comprising:

| Component | mg/L |
|---|---|
| L-Monosodium Glutamate | 10,710 |
| L-Proline | 240 |
| NaCl | 2,500 |
| KCl | 200 |
| KH₂PO₄ | 5,000 |
| MgCl₂.6H₂O | 1,000 |
| Tris base | 1,525 |
| CaCl₂.2H₂O | 20 |
| Hydrolyzed potato starch | 1,000 |

All components were dissolved in water except for the CaCl₂.2H₂O and the hydrolyzed starch. The solution was adjusted to pH 7.6 with a 20% solution of hydrochloric acid. The calcium chloride was added as a 1% solution followed by the starch solution (5%). The resulting solution was adjusted to its final volume with distilled water and the pH checked and adjusted, as required. This solution then was heat sterilized.

A growth factor solution also was used for the fermentation, comprising:

| Component | mg/L |
|---|---|
| L-Cysteine | 4,000 |
| Nicotinic acid | 400 |
| Ascorbic acid | 2,000 |
| Glutathione (reduced) | 10,000 |
| FeSO₄.7H₂O | 1,000 |

The L-cysteine was dissolved in 100 ml of distilled water containing 10 mL concentrated hydrochloric acid. The other growth factors were added to this solution, which then was filter sterilized.

Freeze dried B. pertussis strains 10536 and 1494 were recovered in modified Stainer-Scholte (SS) medium and grown on Bordet-Gengou plates for 3 to 4 days at 34° to 37°C. Growth from the plates was inoculated into a seed flask containing the modified Stainer-Scholte medium and 1% V/V growth factor solution. Growth for 20 to 24 hours at 34° to 37°C was effected on an orbital shaker. The seed flask culture was inoculated into 20 litre seed bottles containing 10 litres of the modified Stainer-Scholte medium and growth factors. This culture was grown for 18 to 30 hours at 34° to 37°C with aeration.

During production of seed fermentor, the fermentor containing modified Stainer-Scholte medium was sterilized by autoclaving. Growth factor solution was sterilized by filter sterilization and added separately. The seed fermentor was inoculated with culture from the seed bottles. Growth was continued for approximately 24 hours at 35° to 37°C with aeration and vortex stirring. pH was maintained at pH 7.0 to 7.5 by adding phosphoric acid using an automatic pH controller.

The production fermentor was inoculated with the culture from the seed fermentor. The production fermentor was grown for 40 hours at 35 to 37°C with surface aeration and vortex stirring. The pH was maintained in the range pH 6.8 to 7.6 by adding a solution of dilute hydrochloric acid containing glutamic acid (2 M glutamic acid : 1 M HCl) using a sterilizable pH electrode and an automatic pH controller.

The fermentation was completed when the culture reached 40 international Opacity Units of pertussis (OU)/ml. Formalin was added to the fermentor contents to a final concentration of 0.2% formaldehyde. The fermentor was incubated at 35 to 37°C for 72 to 88 hours, then cooled and stored at 2° to 8°C.

The formaldehyde treated culture was concentrated by centrifugation. The culture fluid was discarded. The centrifuged cells were resuspended in sterile saline. Thimerisol was added to a final concentration of 0.005% for pertussis vaccine. The detoxified cell suspension was adsorbed onto aluminium phosphate to make pertussis vaccine.

The pertussis vaccine prepared as described above, which is an aqueous preparation of killed whole Bordetella pertussis (lot No. 3082 and 3083A combined) bacteria, was tested for its potency. The mouse potency test for pertussis is carried out according to current FDA (U.S.A.) regulation 21 CFR 620.4. The test determines the relative protection of a test sample as compared to the U.S. standard pertussis vaccine. The WHO (World Health Organization) mouse potency test was performed according to the standard procedure (WHO technical report 638/1979, 760/1987, Annex 4: 800/1990). The weight range of mice was 12.0 to 14.0 gm. The number of mice used per dose were 24. Dose of injection was 0.5 ml I.P. (intra perineal). Dose of injection of challenge was 0.03 ml I.C. (intra cerebral).

The potency for the whole cell vaccine was determined to be 11.4 by the mouse potency test and 3.6 by the WHO test and showed that the whole cell vaccine produced by the present invention may be used as a standard vaccine for pertussis.

The pertussis vaccine procedure described above was repeated again using the B. pertussis strain 10536 and 1494 but to a higher cell mass. The killed whole cell vaccines were separately tested rather than combined for testing and the US standard and WHO mouse potency tests carried out on the vaccines.

The results obtained and summarized in Table III below. The potency data shown in Table III shows that the whole cell vaccine produced by the present invention may be used as a standard vaccine for protection against disease caused by infection by B. pertussis.

### SUMMARY OF DISCLOSURE

In summary of this disclosure, the present invention effects fermentation of Bordetella organism in a unique way to achieve high cell yield and antigen production. Modifications are possible within the scope of this invention.

**TABLE I**

| Growth and antigen production in glutamic acid pH control fermentation for pertussis vaccine production. | | | | |
|---|---|---|---|---|
| Experiment # | Growth O.D.₆₀₀ | PT mg/l | FHA mg/l | 69KD mg/l |
| 1 | 6.33 | 35.65 | 68.15 | 9.20 |
| 2 | 7.75 | 43.84 | 68.40 | 10.10 |
| 3 | 6.44 | 28.80 | 112.10 | 7.80 |
| 4 | 8.00 | 26.17 | 84.70 | 7.40 |

**TABLE II**

| Comparison of the glutamic acid HCl pH control system with standard fermentation for pertussis antigen production. | | |
|---|---|---|
| | Glutamic acid HCl pH control (Method described in Example 1) | Fed-batch Fermentation. (Method described in Example 2) |
| pH controlled by | Glutamic Acid/HCl | Phosphoric acid |
| Duration of Fermentation | 35 - 40 hrs | 45 - 50 hrs |
| PT production mg/L | 28.71 | 20.54 |
| PT production mg/L/O.D | 3.95 | 2.26 |
| 69Kd production mg/L | 7.82 | 5.57 |
| 69Kd production mg/L/O.D | 1.08 | 0.61 |
| FHA production mg/L | 81.3 | 126.5 |
| FHA production mg/L/O.D | 11.21 | 13.93 |
| Growth O.D ₆₀₀ | 7.26 | 9.08 |

The results are given average of more than 6 runs of each type.

**TABLE III**

| Potency Data | | | | |
|---|---|---|---|---|
| Lot # | Strain | OU/ml | Potency | |
| | | | WHO | US |
| 3082/3083A | 1494 + 10536 | 40 | 3.6 | 11.4 |
| 3107B | 1494 | 56 | 6.0 | 18.0 |
| 3108B | 10536 | 62 | 6.5 | 19.5 |

## Claims

1. A method for batch growth of a species of Bordetella, which comprises:
growing said species under aerobic conditions in a liquid culture medium containing nutrients for said growth,
continuously determining the pH of the culture medium during growth of said species, and, in response to said pH determination, simultaneously feeding, as required:
(a) an acid in a concentration sufficient to maintain the pH of the culture medium within a predetermined range of values during growth of said species, and
(b) a nutrient medium for growth of said species containing organic carbon and organic nitrogen.

2. The method claimed in claim 1 wherein said predetermined range of pH values is from about 6.0 to about 9.0.

3. The method claimed in claim 2 wherein said predetermined range of pH values is from about 6.8 to about 7.3.

4. The method claimed in any one of claims 1 to 3 wherein said acid is a mineral acid and said nutrient medium comprises an amino acid, a neutral salt of an amino acid or a mixture thereof.

5. The method claimed in claim 4 wherein said neutral salt of an amino acid is monosodium glutamate.

6. The method claimed in claim 4 wherein said amino acid is glutamic acid.

7. The method claimed in any one of claims 1 to 6 wherein said nutrient medium is dissolved in said acid and said simultaneous feeding is effected by feeding the solution resulting from such dissolution to said culture medium.

8. The method claimed in any one of claims 1 to 7 wherein the mineral acid is hydrochloric acid, phosphoric acid or sulfuric acid.

9. The method claimed in claim 8 wherein said mineral acid is hydrochloric acid and said amino acid is glutamic acid.

10. The method claimed in claim 9 wherein the solution comprises 2 M glutamic acid dissolved in 1 M hydrochloric acid.

11. The method claimed in any one of claims 1 to 10 wherein said Bordetella species is Bordetella pertussis.

12. The method claimed in claim 11 including subsequently isolating Bordetella pertussis antigens from the culture medium and formulating the isolated antigens as an acellular vaccine.

13. The method claimed in claim 11 including subsequently detoxifying whole cells of Bordetella pertussis and formulating the detoxified whole cells as a vaccine.
